# EUROPEAN PATENT APPLICATION

(11) **EP 4 317 414 A1**
(43) Date of publication of application: **07.02.2024**
(21) Application number: 22781670.9
(22) Date of filing: 31.03.2022
(51) Int. Cl.: C12N 1/20, C12N 9/02, C12N 9/14, C08J 11/10, B09B 3/60, C12R 1/38, C12R 1/01

(54) **NOVEL MICROBE WITH PLASTIC DECOMPOSITION ACTIVITY AND USE THEREOF**

(30) Priority: 02.04.2021 KR 20210043089
(71) Applicant: Industry Foundation of Chonnam National University, Gwangju 61186 (KR)
(72) Inventor: YEOM, Soo Jin, Gwangju 61182 (KR); YOON, Chul Ho, Sejong 30152 (KR); CHI, Won Seok, Gwangju 61040 (KR); JO, Jin Hui, Gwangyang-si Jeollanam-do 57806 (KR); KIM, Ye Bin, Gwangju 61174 (KR)
(74) Representative: dompatent von Kreisler Selting Werner - Partnerschaft von Patent- und Rechtsanwälten mbB
(86) International application number: PCT/KR2022/004654
(87) International publication number: WO 2022/211551

(57) **Abstract**

The present disclosure relates to a novel microorganism with plastic (polystyrene)-degrading activity and its use, and newly discovered strains of *Pseudomonas lini* JNU 01 and *Acinetobacter johnsonii* JNU 01 capable of degrading plastic, especially polystyrene, and has the effect of providing a method for degrading plastic using the same.

## Description

### [Technical Field]

The present disclosure relates to a novel microorganism with plastic-degrading activity and its use.

### [Background Art]

Waste includes a variety of materials such as metal, glass, and plastic. Plastics account for a much larger amount of industrial and household waste than metals, and as a result, their value exceeds metals when converted into economic value. Meanwhile, because of the possible depletion of petroleum, the main raw material for plastics, the need for recycling plastic discharged as waste is increasingly emerging.

As various types of plastic are usually used in one product, when recycling plastic, various types of plastic are collected and recycled at once. For this reason, it is difficult for each plastic material to perfectly maintain its purity as a single material because other plastic materials act as impurities. As a result, the quality is poor and the economic value is evaluated to be about 34% lower than that of newly-produced plastic. In particular, it is difficult to economically separate mixed waste plastic in household waste by property, so there is an increasing need to develop technology to sort mixed waste plastic. The plastic recycling process largely consists of a pretreatment process and a recycling process, and during the pretreatment process, mixed waste plastic is separated and sorted according to property. The wet flotation selection method is mainly used to separate and sort mixed waste plastic by property, and however, the wet flotation selection method requires a large amount of water, and even after undergoing the wet flotation selection, about 2% of other materials are mixed in, which leads to low-grade plastic recycling. In particular, polyvinyl chloride (PVC) acts as a foreign substance for plastics that have undergone the wet flotation selection, so prior removal of PVC is essential during the pretreatment process of the plastic recycling process.

Meanwhile, biological technology has recently been developed to treat plastic contained in wastewater or waste using microorganisms. For example, Korea Patent No. 10-0350928 discloses *Klebsiella pneumoniae* CJ-PVA a (Accession No. KFCC-11126), a novel microorganism that grows well under aerobic conditions and has an improved ability to degrade polyvinyl alcohol, and a method for treating wastewater containing polyvinyl alcohol using the same. Additionally, Korea Patent No. 10-0513931 discloses *Microbacterium barkeri* LC (Accession No. KCCM 10507) and a method for biologically degrading polyvinyl alcohol using the same.

The inventors of the present disclosure have completed the present disclosure as a result of research on strains capable of degrading plastic.

### [Summary of Invention]

### [Technical ProblemJ

An aspect of the present disclosure is to provide a strain of *Pseudomonas lini* JNU 01 (Accession No. KCTC 14425BP) having plastic-degrading activity.

Another aspect of the present disclosure is to provide a strain of *Acinetobacter johnsonii* JNU 01 (Accession No. KCTC 14426BP) having plastic-degrading activity.

Still another aspect of the present disclosure is to provide a method for degrading plastic including culturing the aforementioned strain in a plastic-containing medium.

### [Solution to ProblemJ

An aspect of the present disclosure provides a strain of *P. lini* JNU 01 (Accession No. KCTC 14425BP) having plastic-degrading activity.

In an embodiment of the present disclosure, the plastic may include any one or more of polyethylene terephthalate (PET), polyvinyl chloride (PVC), polystyrene (PS), polypropylene (PP), and polyethylene (PE).

Another aspect of the present disclosure provides a strain of *A. johnsonii* JNU 01 (Accession No. KCTC 14426BP) having plastic-degrading activity.

In an embodiment of the present disclosure, the plastic may include any one or more of polyethylene terephthalate (PET), polyvinyl chloride (PVC), polystyrene (PS), polypropylene (PP), and polyethylene (PE).

In an embodiment of the present disclosure, the plastic-degrading activity may be caused by any one or more of alkane monooxygenase (AlkB), alcohol dehydrogenase (Adh), homoserine dehydrogenase (HDH), and S-formylglutathione hydrolase (SGT).

Still another aspect of the present disclosure provides a method for degrading plastic including culturing the above-mentioned strain in a plastic-containing medium.

Yet another aspect of the present disclosure provides a composition for degrading plastic including the above-mentioned strain.

### [Advantageous Effects of Invention]

The present disclosure has the effect of providing strains of *P. lini* JNU 01 (Accession No. KCTC 14425BP) and *A. johnsonii* JNU 01 (Accession No. KCTC 14426BP), which have plastic-degrading activity.

Additionally, the present disclosure has the effect of providing a novel method for degrading plastic using the above-mentioned strain.

### [Brief Description of Drawings]

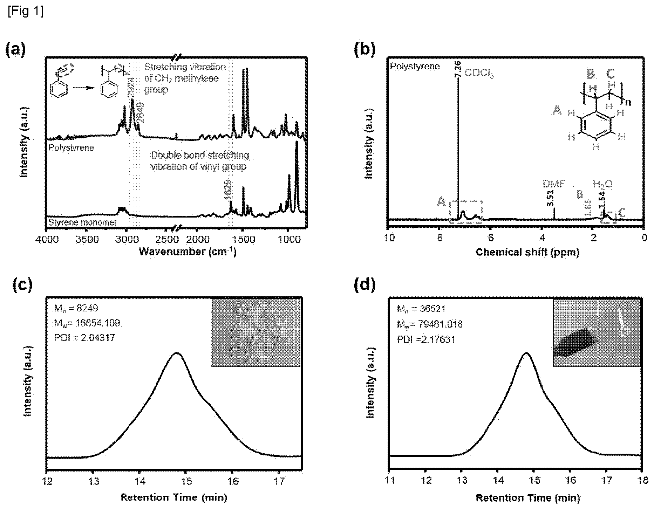

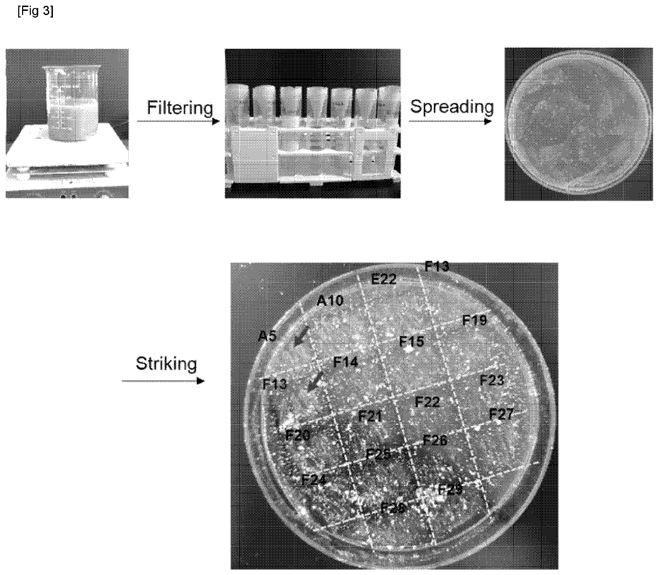

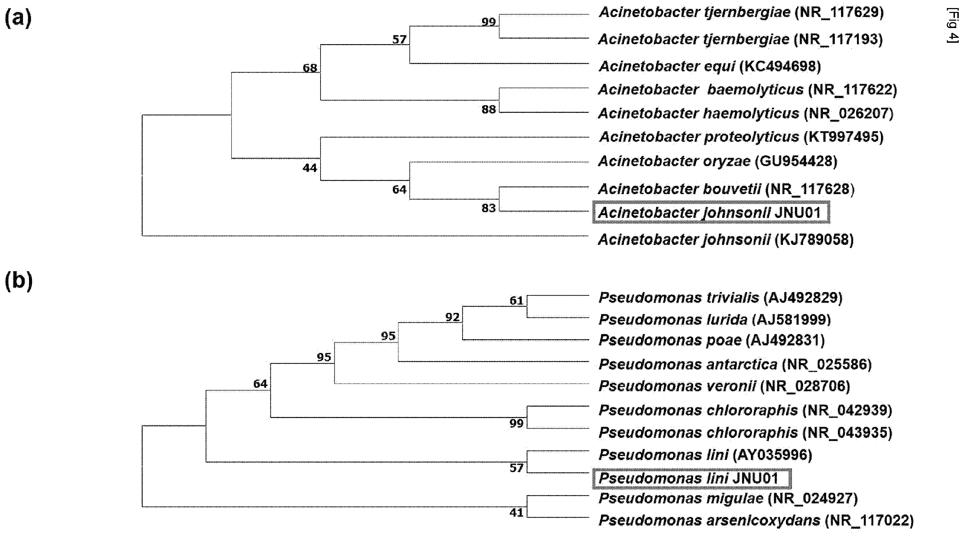

FIG. 1 shows the results of analysis of manufactured low-molecular-weight and high-molecular-weight polystyrene samples, wherein FIG. 1(a) represents FT-IR spectra of styrene and polystyrene after radical polymerization reaction, FIG. 1(b) represents ¹H NMR spectrum of polystyrene in CDCl₃, FIG. 1(c) represents GPC data of low-molecular-weight polystyrene powder, FIG. 1(d) represents GPC data of high-molecular-weight polystyrene film. The inset photos in FIGS. 1(c) and 1(d) are photos of polystyrene powder and film.

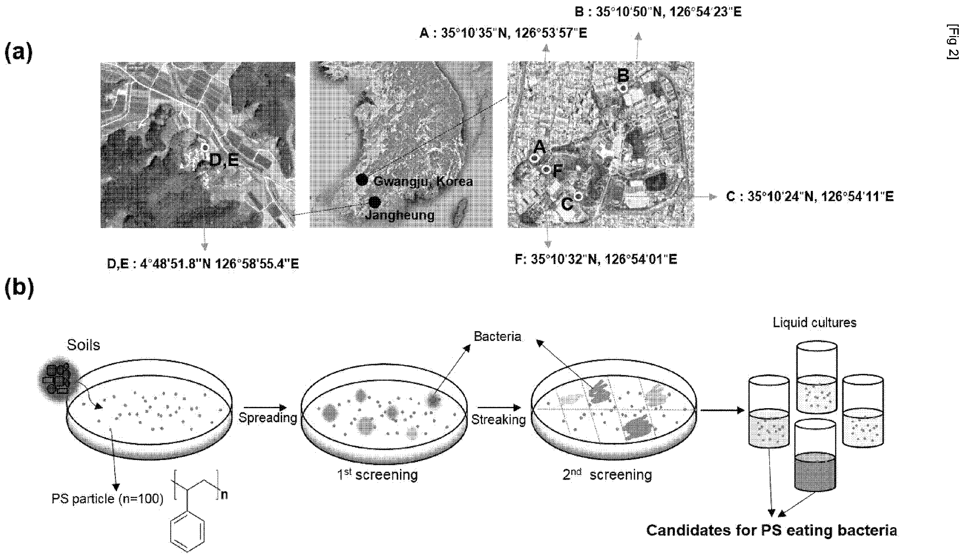

FIGS. 2 to 4 represent the results of isolation and identification of bacterial strains, and FIG. 2 represents the analysis process of the samples and the locations of the sampled soils. Specifically, FIG. 2(a) is a map showing the locations of barn soil collection in Chonnam National University in Gwangju, Korea, and Jangheung where samples A to F were collected, wherein samples A, B, C, and F were obtained from Chonnam National University, and samples D and E were obtained from barns. FIG. 2(b) shows the process of screening microorganisms from soil, and the process is for selection of bacterial candidates that degrade polystyrene, wherein after two selections, a candidate sample was selected in the third experiment. BSM was used in culture, and only PS was used as a carbon source.
And, FIG. 3 shows the procedure for screening microorganisms from soil, wherein microorganisms dispersed in each soil were mixed using a PBS buffer, filtered with whatman paper, and then spread directly onto a BSM solid medium plate using 1 g/L of polystyrene powder as the sole carbon source. After 7 days of culture at 28°C, some colonies were cultured, and re-culture was confirmed even when streaked on a BSM solid medium plate containing 1 g/L of polystyrene powder. Red arrows indicate cells grown on PS plates.
Additionally, FIG. 4 represents a phylogenetic tree derived from comparative analysis of rRNA sequences, which is based on 16sRNA analysis. Specifically, it shows the phylogenetic tree between 16S rRNA gene sequences retrieved from GenBank, and the degree of relatedness was calculated using CLUSTALW in Genome Net, and the phylogenetic tree was calculated with Molecular Evolutionary Genetics Analysis Version X 10.1 (MEGA X 10.1). (a) A5 bacteria (*A. johnsonii* JNU 01), (b) F13 bacteria (*P. lini* JNU 01).

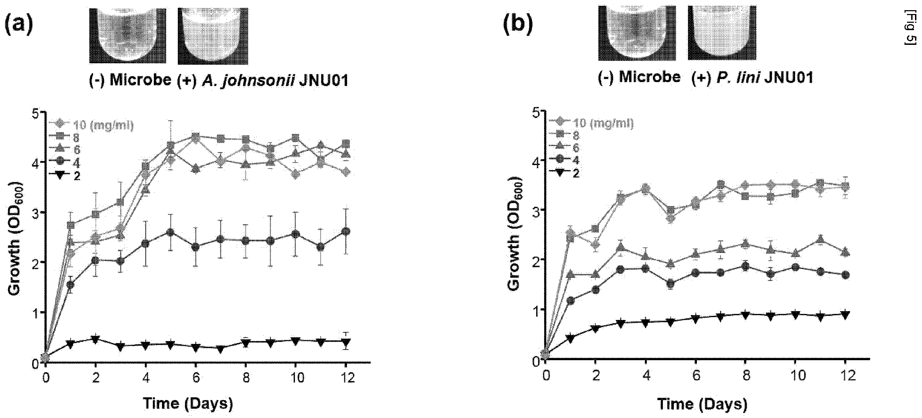

FIG. 5 shows the results of confirming the growth curve of the strains of the present disclosure and their ability to degrade polystyrene, wherein in relation to the growth curve of the strain according to a single carbon source, the OD value (Optical Density) of each sample changed for 12 days when being cultured in a flask containing 10 mL of liquid medium to which polystyrene was added. Each line was distinguished by the concentration of polystyrene (5 points, in the case of polystyrene of 2 mg/ml (filled inverted triangle), 4 mg/ml (filled circle), 6 mg/ml (filled triangle), 8 mg/ml (filled square), and 10 mg/ml (filled diamond). (a) *A. johnsonii* JNU 01, (b) *P. lini* JNU 01.

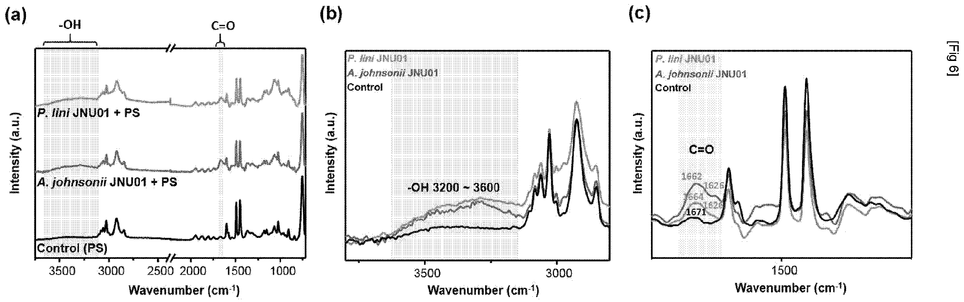

FIG. 6 shows, as the results of FT-IR analysis of low-molecular-weight polystyrene powder, (a) total wavelength, (b) hydroxyl (-OH) group and (c) FT-IR spectrum of the carboxyl (C=O) group of pure polystyrene and degraded polystyrene by *Acinetobacter johnsonii* JNU 01 and *P. lini* JNU 01.

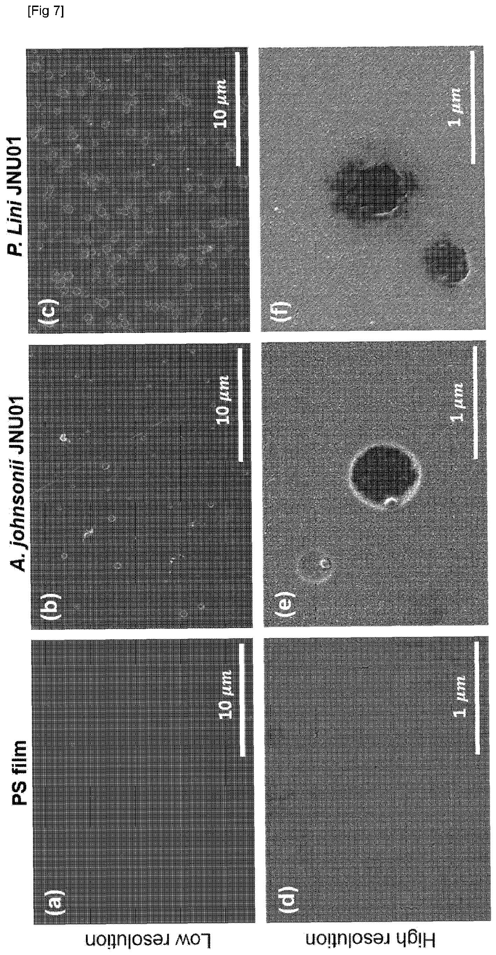

FIG. 7 shows, as SEM images of a high-molecular-weight polystyrene film, FE-SEM images of pure polystyrene (A: low resolution, D: high resolution), and polystyrene degraded by microorganisms *A.r johnsonii* JNU 01 (B: low resolution, E: high resolution) wj and *P. lini* JNU 01 (C: low resolution, F: high resolution).

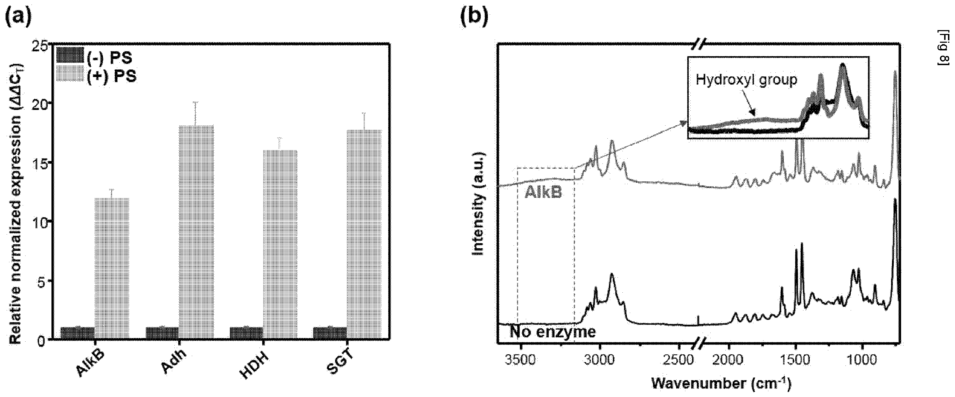

FIG. 8 shows, as the results of sequence analysis and transcriptional expression profiling of candidate genes involved in polystyrene biodegradation of the strain of the present disclosure, the results of testing the expression level of target genes selected from *A. johnsonii* JNU 01. Specifically, the relative expression levels of four different kinds of enzymes measured by RT-qPCR were shown, and the expression levels of four kinds of enzymes related to the degradation of polystyrene, AlkB, Adh, HDH, and SGT, were confirmed by comparing the RNA expression level of RNA expressed in the growth of *A. johnsonii* JNU 01 (control) cultured in BSM with added sodium acetate and that of *A. johnsonii* JNU 01 cultured in BSM with added polystyrene.

### [Best Model

An aspect of the present disclosure provides a strain of *P. lini* JNU 01 (Accession No. KCTC 14425BP) having plastic-degrading activity.

Additionally, an aspect of the present disclosure provides a strain of *A. johnsonii* JNU 01 (Accession No. KCTC 14426BP) having plastic-degrading activity.

The strains of *P. lini* JNU 01 and *A. johnsonii* JNU 01 are selected from the strains which are isolated from the soil where plastic waste was dumped, and are grown after inoculation on a medium containing polystyrene as the sole carbon source.

In an embodiment of the present disclosure, the plastic(s) may be any one or more of polyethylene terephthalate (PET), polyvinyl chloride (PVC), polystyrene (PS), polypropylene (PP), and polyethylene (PE), and may be, more specifically, polystyrene (PS).

In an embodiment of the present disclosure, for the strain of *A. johnsonii* JNU 01 (Accession No. KCTC 14426BP), the plastic-degrading activity may be caused by any one of alkane monooxygenase (AlkB), alcohol dehydrogenase (Adh), homoserine dehydrogenase (HDH), and S-formylglutathione hydrolase (SGT), and more specifically, alkane monooxygenase (AlkB). It can be seen that the above-mentioned four types of enzymes are related to polystyrene degradation in combination or alone based on the fact that the expression levels of these enzymes increased 10 times or more when cultured in a medium containing polystyrene, and particularly, in the case of AlkB, when it was introduced into another strain and the introduced recombinant strain was cultured in a medium containing polystyrene, the residue of the hydroxyl group, which is a product of polystyrene degradation, was identified, so it can be seen that AlkB is related to polystyrene degradation ability.

Additionally, an aspect of the present disclosure provides a method for degrading plastic including culturing the strain in a plastic-containing medium.

Specifically, the strain may be any one or more of the *P. lini* JNU 01 strain and the *A. johnsonii* JNU 01 strain, and a recombinant strain into which AlkB is introduced can also be used.

The plastic may include any one or more of polyethylene terephthalate (PET), polyvinyl chloride (PVC), polystyrene (PS), polypropylene (PP), and polyethylene (PE), and may be, more specifically, polystyrene (PS).

The plastic-containing medium may further contain known materials necessary for culturing strains in addition to the plastics described above, but it may be intended to contain no carbon sources other than plastic when considering the purpose of the present disclosure.

As described above, the strain of the present disclosure has the ability to degrade plastic, particularly polystyrene, due to enzymes such as AlkB, and therefore, when being cultured in a plastic-containing medium, the strain located on the surface of polystyrene degrades polystyrene into compounds with hydroxyl groups through metabolic activity to form pores on the surface of polystyrene.

Another aspect of the present disclosure provides a composition for degrading plastic including the above-mentioned strain.

### [Description of Embodiments]

Hereinafter, one or more embodiments will be described in more detail through examples. However, these examples are intended to illustrate one or more embodiments, and the scope of the present disclosure is not limited to these examples.

### Example 1: Experimental method

### 1.1 Chemicals and Media

NB (nutrient broth) medium (peptone 5.0 g, beef extract 3.0 g/L)), BSM (basal salt medium) (12.8 g Na₂HPO₄·7H₂O, 3 g KH₂PO₄, 0.5 g NaCl, 1 g NH₄Cl, 2 mM MgSO₄, 0.1 mM CaCl₂, per later), styrene (*ReagentPlus*^{®}*,* contains 4-tert-butylcatechol as stabilizer, ≥99%)) and AIBN (2,2'-azobisisobutyronitrile (AIBN, 98%)) were purchased from Sigma-Aldrich (St. Louis, MO, USA). Methanol (MeOH), DMF (N,N'-dimethylformamide), and THF (tetrahydrofuran (HPLC grade)) were purchased from Deoksan (Ansan, South Korea). All chemicals and solvents were used without further purification.

### 1.2 Synthesis of polystyrene powder and film

Polystyrene was synthesized through free radical polymerization. Specifically, in a 50 mL flask, 6.0 g of styrene was dissolved in 50 mL of DMG solvent at room temperature. Afterwards, AIBN initiator was added to the styrene solution and stirred vigorously until the AIBN was dissolved completely. It was left in nitrogen gas (99.999%) for 30 minutes to remove oxygen from the mixture. Through this process, the radical polymerization reaction time can be increased by creating an inert atmosphere. The polymerization was carried out at 90°C for 16 hours with slow stirring. The polymerized product was precipitated in methanol and washed. The said product was centrifuged at high speed (e.g., 10,000 rpm) and washed with methanol. This washing step was repeated several times until unreacted monomers and residues were removed completely. The polystyrene was dried at 60°C under vacuum conditions to remove residual solvent.

Polystyrene film was prepared by solvent evaporation. Specifically, to prepare a 0.5 wt% polystyrene solution, polystyrene powder (0.3 g) was dissolved in THF solvent (6 mL). The polystyrene solution was stirred for several hours to prepare a homogeneous solution through complete polymer degradation. The polystyrene solution was poured into a glass petri dish, which was covered with perforated aluminum foil to slow down the evaporation rate of the solvent. The glass petri dish covered with aluminum foil was placed in an oven at 50°C overnight. A transparent film was formed after the solvent was completely evaporated. Deionized water was poured into the glass petri dish to easily remove the polystyrene film from the substrate. The separated polystyrene film was dried in an oven at 50°C for a while to remove residual volatile solvent. Finally, the dried polystyrene film was stored in a vacuum oven at 60°C to remove residual moisture.

### 1.3 Isolation of microorganism from soil

Soil samples were collected from soils contaminated with waste around Chonnam National University and an old livestock farm in Jangpyeong-myeon, Jangheung-gun. The soils collected from four locations within Chonnam National University are located in Gwangju, South Korea, as shown in FIG. 2 (Location A 35°10'35"N, 126°53'57"E; Location B 35°10'50"N, 126°54'23"E; Location C 35°10'24"N, 126°54'11"E; Location F 35°10'32"N, 126°54'01"E) . Additionally, the second location is located in Jangheung, South Korea, as shown in FIG. 2 (Locations D and E 34.81438, 126.982044). Approximately 5 g of soil sample was mixed with 100 mL PBS buffer in a 250 mL beaker, and filtered through Whatman paper (pore size 11 um). The filtered solution was spread onto a BSM solid medium plate containing 1 g/L of polystyrene powder, and cultured at 28°C for 7 days. Colonies which had been cultured in polystyrene-containing medium were streaked on a BSM solid medium plate containing 1 g/L polystyrene, and it was checked whether they were cultured on the polystyrene solid medium plate. Then, the cultured bacteria were inoculated into a liquid medium containing 1 g/L of polystyrene powder and cultured at 28°C and 200 rpm for 12 days. And optical density (OD) was measured every day.

### 1.4 Cell growth of bacteria in polystyrene medium

Finally, the selected bacteria were inoculated into liquid nutrient medium, and cultured overnight at 28°C in an incubator with 200 rpm agitation. And it was centrifuged at 3800 rpm for 20 minutes, and washed with BSM. And it was inoculated into a BSM flask containing polystyrene powder, and cultured at 28°C and 200 rpm for 12 days. It was performed at the final polystyrene powder concentration of 2, 4, 6, 8, and 10 mg/ml. Optical density (OD) was measured with a UV-Vis Spectrophotometer (SHIMADZU, Kyoto, Japan).

### 1.5 Identification of bacteria strain

Twenty individual colonies were obtained from the above-mentioned cultured bacteria (A-F depending on collection location) by streaking them onto an NB solid medium plate (beef Extract 3.0 g, peptone 5.0 g, agar 15.0 g in 1 L of deionized water, pH 6.8). Individual DNA extraction of each colony was performed using the HiGene Genomic DNA Prep Kit (BIOFACT, Daejeon, South Korea). 16sRNA analysis was performed to identify the bacterial species. Universal primers 785F (GGATTAGATACCCTGGTA) and 907R (CCGTCAATTCMTTTRAGTTT) were used for sequencing (Solgent, Daejeon, South Korea).

### 1.6 Analysis of chemical structure, physical properties and morphological analysis of polystyrene

The selected bacteria were inoculated into a liquid medium containing polystyrene powder and polystyrene film (0.6 cm X 0.4 cm), and cultured at 28°C and 200 rpm for 7, 14, and 30 days. After incubation, a 2% SDS solution was used for 4 hours, and then the polystyrene powder and polystyrene film were washed three times with 10 mL of deionized water and MeOH. The washed polystyrene powder and film were completely dried in a vacuum oven at 60°C, and measured by FT-IR, GPC, and SEM. ¹H NMR analysis was measured at 25°C using a Nuclear Magnetic Resonance Spectrometer (Unity INOVA 500, 500 MHz) at Korea Basic Science Institute (KBSI, Gwangju, South Korea). The NMR sample solution was prepared as a 0.5% (w/v) solution using CDCl₃ solvent. Chemical shift was measured in ppm (parts per million) based on the residue peak of CDCl₃ (7.26 ppm). FT-IR (Fourier-transform infrared spectroscopy, IRAffinity-1S, Shimadzu, Kyoto, Japan) was used to measure functional groups of polystyrene samples in the wavelength range of 4000-500 cm⁻¹ with a resolution of 4 cm⁻¹. M_{w}, Mₙ (The weight-averaged and number-averaged molecular weights), and PDI (polydispersity index) of the polystyrene sample were evaluated based on standard columns including a guard column (Shimadzu, GVP-ODS), Phenogel linear column (Phenomenex, pore size range 100 - 100000 Å), and Phenogel column (Phenomenex, pore size of 100 Å) using a gel permeation chromatography (gel permeation chromatography, GPC) device (Shimadzu Prominence LC-20A). The GPC sample solution was prepared by dissolving 20 mg of polystyrene in 4 mL of THF (HPLC grade) to prepare a 0.5% (w/v) solution. The surface shape of the polystyrene film was observed using a FE-SEM (filed emission scanning electron microscope, Hitachi SU-70) with an acceleration voltage of 15 kV at the Korea Basic Science Institute (KBSI) in Gwangju. Before FE-SEM measurements, a conductive layer was directly formed on the surface of the polystyrene film using platinum material of 20 mA for 100 s. This platinum coating allows for FE-SEM measurement of polystyrene polymer samples.

### 1.7 Q-RT-PCR

To confirm the expression profile of polystyrene biodegradation enzymes, previously reported genome sequence data of A. *johnsonii* (GenBank Accession No. CP022298.1) were used. A. *johnsonii* JNU 01 was cultured in BSM medium supplied with polystyrene and sodium acetate as the sole carbon source in the mid-exponential stage. Total RNA was isolated using the ReliaPrepTM RNA Cell Miniprep System (Promega, Madison, Wisconsin, USA), and RNA extraction was performed according to the manufacturer's instructions. After quantification of total RNA, 1 µg of RNA was used with 4 µL of 5X iScript Reaction Mix (Bio-rad, Hercules, CA, USA), 1 µL of iScript Reverse Transcriptase (Bio-rad), and various volumes of nuclease-free water. cDNA synthesis was performed at 25°C for 5 minutes, reverse transcription was performed at 46°C for 20 minutes, and RT inactivation was performed at 95°C for 1 minute. In RT-qPCR, the CFX Connect Real-Time PCR Detection System (Bio-rad) was used to measure the expression level of each gene for each primer set (Table 1). Each primer set was constructed based on the genomic DNA of *A. johnsonii* strain IC001 (Genebank Accession No. CP022298.1). Then, 5 µL of cDNA diluted 1/5 was mixed with iQTM SYBR Green Supermix (Bio-rad) . 16s rRNA was used as an endogenous control. The relative quantification of the expression of each gene in the test group compared to the control group was calculated using the Bio-rad CFX Maestro 2.0 program, and the expression level of each gene was measured as the relative value between the test group and the control group.

**[Table 1]**

| **Target Enzyme** | **Forward** | **Reverse** |
|---|---|---|
| Alkane 1-monooxygenase(AlkB ) | 5'-AGCGTGGTCGTCAAAAATGC-3 ' | 5'-GCACCGACCAGACCATCTAC -3' |
| Alcohol dehydrogenase (Adh) | 5'-CAGGCTGTACCGATGGTCTT-3 ' | 5'-TGCCTGTTGCCACTCTAGTT -3' |
| Homoserine dehydrogenase (HDH) | 5'-CGAAGATGGTGCTGGAACGA-3 ' | 5'-GCGTGGTTGTTGCATGATGG -3 ' |
| S-Formylglutathione hydrolase (SGT) | 5'-CAGATCAAGCTGAGTGGCTGA -3' | 5'-GTCATAGCCTGCATGTTCGC -3' |
| 16s rRNA | 5'-AGATGGATTGGTGCCTTCGG-3 ' | 5'-AGGGCCATGATGACTTGACG -3' |

### Example 2: Experimental results

### 2.1 Synthesis of low-molecular-weight and high-molecular-weight polystyrene samples

Low-molecular-weight polystyrene was synthesized through radical polymerization using a AIBN initiator of a high concentration. FT-IR of low-molecular-weight polystyrene and styrene monomer was confirmed (FIG. 1(a)). The FT-IT spectrum of styrene monomer shows stretching vibration of the double bond of the vinyl group at 1692 cm⁻¹, which may be the active site for polymerization. After the radical polymerization reaction, the characteristic peak of the vinyl group at 1692 cm⁻¹ disappeared, indicating a successful polymerization reaction. Additionally, in the FT-IR spectrum of low-molecular-weight polystyrene, two characteristic peaks of asymmetric and symmetric stretching vibrations of the =CH₂ methylene group were identified at 2924 and 2849 cm⁻¹. This means that -CH₂ methylene group is formed through a successful polymerization reaction. Meanwhile, the FT-IR spectrum for low-molecular-weight polystyrene showed three characteristic peaks at 3082, 3062, and 3025 cm⁻¹ due to directional C-H stretching vibration. Additionally, the absorption band of C-C bond was identified at 1600 - 1450 cm⁻¹. In particular, the weak peaks at 1603 and 1579 cm⁻¹ reflect the C-C stretching vibration of the planar aromatic ring, while the strong peaks at 1495 and 1453 cm⁻¹ reflect the C-C stretching vibration in the aromatic ring. In the low wavenumber region, characteristic peaks of in-plane and out-of-plane C-H bending of the aromatic ring can be identified. The weak peaks at 1069 and 1028 cm⁻¹ correspond to the in-plane C-H bending of the aromatic ring, and the strong peak at 759 cm⁻¹ corresponds to the out-of-plane C-H bending of the aromatic ring, which is attributed to the aromatic substitution pattern. The inherent properties of low-molecular-weight polystyrene indicate that polystyrene is successfully formed through radical polymerization without any characteristic impurities. FIG. 1(b) shows the ¹H NMR spectrum of low-molecular-weight polystyrene powder. For reference, low-molecular-weight polystyrene powder was dissolved in chloroform-d solvent (0.5 w/v%). Two sharp peaks were identified in the ¹H NMR spectrum of low-molecular-weight polystyrene powder, indicating 7.28 ppm of chloroform and 1.54 ppm of water. The low-molecular-weight polystyrene powder was completely dried in a vacuum oven, but some water molecules were introduced during the use of the experimental equipment. The ¹H NMR spectrum of low molecular weight polystyrene powder shows three specific peaks of protons. First, the characteristic peak at 6.2-7.2 ppm, marked with letter "A" (orange box) in FIG. 1(b), is due to the protons of the aromatic ring. Second, the characteristic peak at 1.85 ppm, marked with letter "B" in FIG. 1(b) (green), is due to a proton near the carbon to which the aromatic group is attached in the linear chain. Third, the peak at 1.44 ppm, marked with letter "C" (blue) in FIG. 1(b), is due to the proton of the backbone away from the aromatic ring. The ¹H NMR spectrum shows that low-molecular-weight polystyrene powder was well synthesized through radical polymerization using an AIBN initiator.

To prepare low- and high-concentration polystyrene samples, radical polymerization reactions using high- and low-concentration initiators were performed, respectively. Through the same measurement method (FT-IR and NMR) as described above, it was confirmed that the high-molecular-weight polystyrene sample was also successfully polymerized. Additionally, GPC measurements were performed to confirm the molecular weight of the low-molecular-weight and high-molecular-weight polystyrene powders/films in FIGS. 1(c) and 1(d). It was confirmed that the M_{w} of the low-molecular-weight polystyrene powder was 16854 and the Mₙ thereof was 8249, which showed a PDI of 2.04. The inset in FIG. 1(c) represents polystyrene powder. The photo shows that low-molecular-weight polystyrene powder has a low molecular weight and thus low mechanical strength. Due to the low physical strength, a strong film could not be formed using low-molecular-weight polystyrene powder. Therefore, the high-molecular-weight polystyrene was synthesized through a radical polymerization reaction using a relatively low concentration of initiator. The high-molecular-weight polystyrene film was confirmed to have M_{w} of 79481, Mₙ of 36521, and PDI of 2.17. The inset photo in FIG. 1(d) shows the polystyrene film formed by the high-molecular-weight polystyrene sample. Polystyrene film was used for SEM surface observation, and polystyrene powder was used in the remaining experiments.

### 2.2 Isolation and identification of bacteria strains

Strains for selecting polystyrene degrading strains were obtained from six locations (A, B, C, D, E, F) where plastic waste and waste were buried in the landfill. Soil samples were collected from waste landfills and plastic landfills in A, B, C, and F located at Chonnam National University. Samples at locations D and E were collected from soil samples from beneath old diesel barrels from a small farm. Specific collection locations and selection methods are described in FIG. 2(b). Specifically, low-molecular-weight polystyrene powder was synthesized in the first initial selection system. Next, each soil was dissolved in PBS buffer and filtered through Whatman paper. Then, it was spread directly onto a BSM solid medium plate with 1 g/L of polystyrene powder as the sole carbon source. After being cultured at 28°C for 7 days, some colonies grew, and were confirmed to re-grow after being streaked on a BSM solid medium plate containing 1 g/L polystyrene powder (FIG. 3). Through this process, two microbial strains were selected among 20 colonies grown on a BSM solid medium plate containing 1 g/L of polystyrene powder. Two microbial strains were successfully grown in BSM medium containing 1.5 g/L of polystyrene powder as the sole carbon source (FIG. 3). The bacteria were named A5 and F13, and were sampled from regions A and F, respectively.

Based on 16s rRNA gene sequences, the two bacterial strains isolated from each region belonged to two genera: Acinetobacter and Pseudomonas. As confirmed in FIG. 4, these include strains of *A. oryzae, A. bouvetii, A. proteolyticus, A. haemolyticus, A. baemolyticus, A. tjernbergiae, A. equi, A. johnsonii, P. lini, P. migulae, Pchlororaphis, P. veronii, P. antarctica, P. arsenicoxydans, P. lurida, P. trivialis,* and *P. poae.* It was confirmed that the 16s rRNA gene sequences of A5 (*A. johnsonii* JNU 01) and F13 (*P. lini* JNU 01) are 99% identical to *A. johnsonii* (Genebank Accession No. NR_117624.1) and *P. lini* (Genebank Accession No. NR_029042.2) (see Table 2).

**[Table 2]**

| **strain** | **Microorganisms** | **%** | **Accession number** |
|---|---|---|---|
| A5 | *Acinetobacter johnsonii* JNU 01 | 99% | NR_117624.1 |
| F13 | *Pseudomonas lini* JNU 01 | 99% | NR_029042.2 |

The *A. johnsonii* JNU 01 strain was deposited at Korea Research Institute of Bioscience and Biotechnology Biological Resources Center (Korean Collection for Type Cultures) under the accession number KCTC 14426BP on January 5, 2021. Additionally, the *P. lini* JNU 01 strain was also deposited at Korea Research Institute of Bioscience and Biotechnology Biological Resources Center (Korean Collection for Type Cultures) under the accession number KCTC 14425BP on January 5, 2021.

### 2.3 Confirmation of growth curve and polystyrene degradation efficiency of A. johnsonii JNU 01 and P. lini JNU 01

*A. johnsonii* JNU 01 and *P. lini* JNU 01 grew well in BSM medium containing polystyrene, which was confirmed by the turbidity of a test tube using polystyrene as a carbon source (FIG. 5). When there were no microorganisms in the BSM medium containing polystyrene, it was confirmed that the medium was transparent.

The degradation and utilization of polystyrene by *A. johnsonii* JNU 01 and *P. lini* JNU 01 were studied by observing cell growth for 12 days in BSM medium containing various concentrations of polystyrene (2 g/L to 10 g/L) (FIGS. 5(a) and 5(b)). *A. johnsonii* JNU 01 began dividing immediately after culture, and reached the rapid growth phase between 1st day and 6th day of the experiment. On the other hand, *P. lini* JNU 01 reached the rapid growth stage between 1st day and 4th day of the experiment. In the case of *A. johnsonii* JNU 01, high levels of cell growth were induced in polystyrene of 4 mg/ml or more. At higher polystyrene concentrations, the *P. lini* JNU 01 strain accumulated more biomass (reflected by OD 600), and needed more time to reach the stationary phase. After 4 days after using 2 g/L to 10 g/L polystyrene, the stationary stage was confirmed in all strains (FIG. 5).

### 2.4 FT-IR analysis of low-molecular-weight polystyrene powder

FT-IR was measured to confirm the chemical structure of the low-molecular-weight polystyrene powder in which the degradation by above-mentioned strains had been performed. FIG. 6(a) represents the FT-IR spectra for a non-degraded polystyrene sample (marked as PS control) and a polystyrene sample biologically degraded by culturing *A. johnsonii* JNU 01 and P. *lini* JNU 01 for 12 days. It was confirmed that the FR-IR spectra for non-degraded polystyrene and biologically degraded polystyrene represented peaks of the same characteristics without noticeable changes. FIG. 6(b) represents FT-IR spectra overlaid with the same baseline for non-degraded polystyrene and polystyrene biologically degraded by *A. johnsonii* JNU 01 and *P. lini* JNU 01 at high wavenumbers. The FR-IR spectrum of polystyrene samples biologically degraded by *A. johnsonii* JNU 01 and *P. lini* JNU 01 shows a weak optical absorption band at 3500-3200 cm⁻¹. This broad peak represents the -OH stretching vibration, indicating the inclusion of hydroxyl groups in the bacterially and biologically degraded polystyrene sample. In particular, the FR-IR spectrum for polystyrene samples shows significantly stronger peak intensities for the -OH stretching vibrations due to significant biological degradation by *A. johnsonii* JNU 01 and *P. lini* JNU 01 (FIG. 5(b)). On the other hand, the polystyrene sample that did not undergo biological degradation did not show a significant -OH characteristic peak. FT-IR spectra show that polystyrene samples may have hydroxyl (-OH) due to biological degradation using *A. johnsonii* JNU 01 and *P. lini* JNU 01. These functional properties offer great potential for degrading polystyrene microplastics into smaller plastic molecules.

### 2.5 SEM image of high-molecular-weight polystyrene film

FIGS. 7(a) to 7(c) show low magnification (low resolution) FE-SEM images of non-degraded polystyrene and biologically degraded polystyrene films by culturing *A. johnsonii* JNU 01 and *P. lini* JNU 01 for 30 days. To remove bacteria attached to polystyrene, a 2% sodium dodecyl sulfate (SDS) solution was used for 4 hours, and washed with distilled water and methanol. FIG. 7(a) shows a top surface SEM image of the outer surface of a non-degraded polystyrene film. Non-degraded polystyrene film has an intact, smooth surface without any deformations (holes or cracks) . On the other hand, it was confirmed that the polystyrene film biologically degraded by *A. johnsonii* JNU 01 and *P. lini* JNU 01 had an overall porous structure (FIGS. 7(b) and 7(c)). In particular, FIG. 7(c) shows that the polystyrene film biologically degraded by *P. lini* JNU 01 exhibited a significant porous form, indicating that the polystyrene film reacted biologically due to significant catalytic activity. To confirm biological degradation by bacteria, high magnification (high resolution) FE-SEM images of non-degraded polystyrene films and polystyrene films biologically degraded by *A. johnsonii* JNU 01 and *P. lini* JNU 01, as shown in FIGS. 7(d), 7(e) and 7(f), were checked. No significant degradation phenomenon was confirmed in the pure polystyrene film (FIG. 7(d)). These results confirmed that no specific microbial reaction occurred in the polystyrene film that was not degraded. Meanwhile, FIGS. 7(e) and 7(f) show the top FE-SEM images of biologically degraded polystyrene films with various bacteria. The biologically degraded polystyrene films exhibited porous structures due to microbial activity. This indicates that microorganisms react biologically with the polystyrene film to cause the surface degradation of the polystyrene film. By including a washing step, microorganisms on the surface of the polystyrene film were removed, and the microbial cells disappeared, but only the biologically formed porous structure remained. FE-SEM images show biological degradation of polystyrene films by *A. johnsonii* JNU 01 and *P. lini* JNU 01.

### 2.6 Sequence analysis and transcriptional expression analysis of candidate genes in A. johnsonii JNU 01 for polystyrene biodegradation

The total genomic DNA of the *A. johnsonii* strain has been reported in GenBank under accession number CP022298.1. It is known that in the biological degradation of long-chain hydrocarbons, alkane monooxygenase (AlkB) can be active on C-H bonds in internal C-C bonds. In the genome sequence of the *A. johnsonii* strain, two genes presumed to encode AlkB and Adh (alcohol dehydrogenase) were found to be involved in the first step of polystyrene degradation. Conventionally, homoserine dehydrogenase (HDH) and S-formylglutathione hydrolase (SGT) are known as the polystyrene biodegradability target gene of Pseudomonas sp. DSM 50071 derived from the intestine of a larvae.

To clarify the contribution levels of the four putative genes for the biodegradation of polystyrene, the transcriptional profiles were examined. The expression levels of AlkB, Adh, HDH, and SGT as candidate genes related to polystyrene degradation, were confirmed to have increased ten or more times in all target genes compared to the control group (FIG. 8(a)). Selective upregulation of specific genes, AlkB, Adh, HDH and SGT, but not a large number of enzymes, was essential to polystyrene degradation for efficient acquisition of energy and cellular components for growth and amplification of *A. johnsonii* JNU 01 strain under nutrient-limited conditions. Since AlkB is an enzyme with a known C-H activation function, it is assumed to contribute to the first step of mineralization of polystyrene. To confirm the function of AlkB in the degradation of polystyrene, AlkB was cloned from *A. johnsonii* JNU 01 into pET28a(+) vector and expressed in *E. coli* C2566 strain. The extract containing the strain was reacted with polystyrene powder for 18 hours, and the polystyrene powder was analyzed by FT-IR. As a result, the polystyrene powder in the reaction mixture was confirmed to have a hydroxyl group, as confirmed in FIG. 8(b) . These results suggest that AlkB contributes to polystyrene degradation through hydroxylation. Future studies of polystyrene degradation by combination with other genes, such as Adh, HDH and SGT, and AlkB are needed.

### Example 3: Conclusion

This study confirmed that bacterial strains such as Acinetobacter and Pseudomonas, isolated from the soil, contribute to the acceleration of polystyrene biodegradation. Bacterial cultures were grown in an aqueous medium containing polystyrene microplastics as the sole carbon source to achieve an optical density (600 nm) of 3 to 4.5. Colony formation on the surface of polystyrene microplastics was observed by SEM, and related with polystyrene microplastic degradation confirmed by FR-IR. These results indicate the confirmation of new function of Acinetobacter and Pseudomonas as strains capable of degrading polystyrene.

So far, the present disclosure has been discussed focusing on its preferred examples. A person skilled in the art to which the present disclosure pertains will understand that the present disclosure may be implemented in a modified form without departing from the essential characteristics of the present disclosure. Therefore, the disclosed examples should be considered from an illustrative rather than a restrictive standpoint. The scope of the present disclosure is not indicated by the foregoing description but in the claims, and all differences within the equivalent scope should be construed as being included in the present disclosure.

### [Accession Number]

Name of depository institution: Korea Research Institute of Bioscience and Biotechnology (KRIBB)
Accession No. KCTC14425BP
Accession date: January 5, 2021
Name of depository institution: Korea Research Institute of Bioscience and Biotechnology (KRIBB)
Accession No. KCTC14426BP
Accession date: January 5, 2021

## Claims

1. A strain of *Pseudomonas lini* JNU 01 (Accession No. KCTC 14425BP) having plastic-degrading activity.

2. The strain of claim 1, wherein the plastic includes any one or more of polyethylene terephthalate (PET), polyvinyl chloride (PVC), polystyrene (PS), polypropylene (PP), and polyethylene (PE).

3. A strain of *Acinetobacter johnsonii* JNU 01 (Accession No. KCTC 14426BP) having plastic-degrading activity.

4. The strain of claim 3, wherein the plastic includes any one or more of polyethylene terephthalate (PET), polyvinyl chloride (PVC), polystyrene (PS), polypropylene (PP), and polyethylene (PE).

5. The strain of claim 3, wherein the plastic-degrading activity is caused by any one or more of alkane monooxygenase (AlkB), alcohol dehydrogenase (Adh), homoserine dehydrogenase (HDH), and S-formylglutathione hydrolase (SGT).

6. A method for degrading plastic, the method comprising culturing a strain according to Claim 1 or Claim 3 in a plastic-containing medium.
